(19) Europäisches Patentamt / European Patent Office / Office européen des brevets

(11) **EP 3 422 935 B1**

(12) **FASCICULE DE BREVET EUROPEEN**

(45) Date de publication et mention de la délivrance du brevet:
**28.10.2020 Bulletin 2020/44**

(21) Numéro de dépôt: **17707337.6**

(22) Date de dépôt: **28.02.2017**

(51) Int Cl.:
*A61B 5/0476* (2006.01)     *A61B 5/048* (2006.01)

(86) Numéro de dépôt international:
**PCT/EP2017/054574**

(87) Numéro de publication internationale:
**WO 2017/148900 (08.09.2017 Gazette 2017/36)**

(54) **PROCEDE DE DETECTION D'ELEMENTS D'INTERET DANS DES SIGNAUX ELECTROPHYSIOLOGIQUES ET DETECTEUR**

VERFAHREN ZUR ERKENNUNG VON BESTIMMTEN ELEMENTEN IN ELEKTROPHYSIOLOGISCHEN SIGNALEN UND DETEKTOR

METHOD FOR DETECTING ELEMENTS OF INTEREST IN ELECTROPHYSIOLOGICAL SIGNALS AND DETECTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.02.2016 FR 1600332**

(43) Date de publication de la demande:
**09.01.2019 Bulletin 2019/02**

(73) Titulaires:
- **Universite d'Aix-Marseille (AMU)**
  **13284 Marseille Cedex 07 (FR)**
- **Institut National de la Santé et de la Recherche Médicale**
  **75013 Paris (FR)**
- **Assistance Publique - Hôpitaux de Marseille (AP-HM)**
  **13005 Marseille (FR)**

(72) Inventeurs:
- **ROEHRI, Nicolas**
  **13008 Marseille (FR)**
- **BENAR, Christian George**
  **13380 Plan de Cuques (FR)**
- **BARTOLOMEI, Fabrice**
  **13360 Roquevaire (FR)**

(74) Mandataire: **Macquet, Christophe**
**Macquet & Associés**
**Arche des Dolines**
**7, rue Soutrane**
**06560 Sophia Antipolis (FR)**

(56) Documents cités:
**US-A1- 2012 245 481**

- **VAN VUGT M K ET AL: "Comparison of spectral analysis methods for characterizing brain oscillations", JOURNAL OF NEUROSCIENCE METHODS, ELSEVIER SCIENCE PUBLISHER B.V., AMSTERDAM, NL, vol. 162, no. 1-2, 15 mai 2007 (2007-05-15), pages 49-63, XP027016052, ISSN: 0165-0270 [extrait le 2007-04-05]**
- **HAMANEH MEHDI BAGHERI ET AL: "Automated Removal of EKG Artifact From EEG Data Using Independent Component Analysis and Continuous Wavelet Transformation", IEEE TRANSACTIONS ON BIOMEDICAL ENGINEERING, IEEE SERVICE CENTER, PISCATAWAY, NJ, USA, vol. 61, no. 6, 1 juin 2014 (2014-06-01), pages 1634-1641, XP011548243, ISSN: 0018-9294, DOI: 10.1109/TBME.2013.2295173 [extrait le 2014-05-15]**
- **AGUSTÃ-N LAGE-CASTELLANOS ET AL: "False discovery rate and permutation test: An evaluation in ERP data analysis", STATISTICS IN MEDICINE, 1 janvier 2009 (2009-01-01), pages n/a-n/a, XP055318732, US ISSN: 0277-6715, DOI: 10.1002/sim.3784**
- **ROMAIN GRANDCHAMP ET AL: "Single-Trial Normalization for Event-Related Spectral Decomposition Reduces Sensitivity to Noisy Trials", FRONTIERS IN PSYCHOLOGY, vol. 2, 1 janvier 2011 (2011-01-01), XP055318485, ISSN: 1664-1078, DOI: 10.3389/fpsyg.2011.00236**

**Description**

DOMAINE DE L'INVENTION

La présente invention concerne un procédé de détection d'éléments d'intérêt dans des signaux électrophysiologiques et, plus particulièrement, dans des signaux intracrâniens. Elle concerne en outre un détecteur pour la mise en œuvre d'un tel procédé.

ART ANTERIEUR

L'électrophysiologie est l'étude des phénomènes électriques et électrochimiques qui se produisent dans les cellules ou les tissus des organismes vivants. Elle implique la mesure de différences de tensions ou de courants électriques à différentes échelles biologiques, d'un canal ionique isolé jusqu'à des organes entiers. En neurosciences, l'électrophysiologie étudie l'activité électrique des neurones ou, plus largement, de l'activité électrique et magnétique du système nerveux, au moyen de l'électro-encéphalographie et de la magnéto-encéphalographie.

L'électroencéphalographie intracérébrale est une méthode invasive d'enregistrement de l'activité du cerveau. Cette méthode est utilisée en neurologie, par exemple dans le cadre d'un bilan pré-chirurgical préparatoire à une opération visant à retirer, par exérèse, des tissus épileptogènes. Analogue à l'électroencéphalographie de surface en ce qu'elle mesure les variations de potentiel électrique dues à l'activité électrophysiologique des neurones, l'électroencéphalographie intracrânienne s'en distingue par le fait que les électrodes sont positionnées soit sur la surface de la pie-mère, sous la dure-mère, soit sous les méninges directement sur la surface corticale (électrocorticographie, ECoG), soit, enfin, à l'intérieur même du tissu cérébral au moyen d'électrodes implantées en profondeur selon une méthode stéréotaxique (stéréo-électroencéphalographie, SEEG).

Les signaux électrophysiologiques sont formés par un mélange d'oscillations à de multiples fréquences et d'activités plus transitoires. Dans les rythmes physiologiques, l'oscillation la plus présente est le rythme alpha, observé en éveil calme. Les marqueurs des différentes phases de sommeil sont des éléments de formes variées. En pathologie, l'électrophysiologie reste la méthode de référence pour observer les décharges épileptiques, pointes ou oscillations hautes fréquences (HFOs - High Frequency Oscillations), dont la fréquence est comprise entre 80 et 500 Hz voire plus. Ces oscillations hautes fréquences peuvent être divisées en trois catégories. Il s'agit des oscillations dites Gamma Hautes (HG, High-Gamma), qui ont une fréquence comprise entre 80 et 150 Hz, des oscillations du type Ondulations Rapide (R, Ripple), qui ont une fréquence comprise entre 150 et 250 Hz), et des oscillations dites Ondulations Très Rapides (FR, Fast Ripple), qui ont une fréquence comprise entre 250 et 500 Hz.

L'analyse visuelle de ces signaux est longue et difficile. En effet, les signaux d'intérêt peuvent être de très faible amplitude. De plus, l'analyse présente une grande variabilité d'un opérateur humain à un autre.

On a donc cherché à développer des procédés de représentation et de détection, ainsi que des détecteurs associés, capables de détecter automatiquement des éléments d'intérêt, tels que des activités transitoires ou des activités oscillatoires, au travers de toute la gamme de fréquences des signaux électrophysiologiques.

Une première difficulté pour un détecteur automatique est que les activités transitoires et oscillatoires se chevauchent en fréquence. Un simple filtrage ne suffit donc pas.

Une solution potentielle, décrite dans le document VAN VUGT M K ET AL: "Comparison of spectral analysis methods for characterizing brain oscillations", JOURNAL OF NEUROSCIENCE METHODS, ELSEVIER SCIENCE PUBLISHER B.V., AMSTERDAM, NL, vol. 162, no. 1-2, 15 mai 2007 (2007-05-15), pages 49-63,ISSN: 0165-0270, est de procéder à une analyse temps-fréquence. Toutefois, cette analyse se heurte à une seconde difficulté, qui provient du fait que les signaux ont une énergie beaucoup plus grande à basse fréquence qu'à haute fréquence (spectre en $1/f^\alpha$).

Une troisième difficulté vient du réglage d'un seuil pour la détection automatique, qui devrait être au maximum indépendant de l'utilisateur humain.

Le document brevet publié sous le numéro US2012/0245481A1 divulgue un détecteur, qui identifie certaines oscillations hautes-fréquences dans un signal encéphalographique de type ECoG. Ce détecteur filtre un signal dont la fréquence est comprise entre 80 et 500 Hz et calcule l'énergie de ce signal dans une fenêtre glissante. Dans une première étape proposée dans ce document, on applique l'algorithme de détection de Staba et al., 2002. Toutes les activités dépassant un certain seuil d'énergie sont alors sélectionnées. Toutefois, cette première étape souffre d'un trop grand nombre de fausses détections. C'est la raison pour laquelle une étape subséquente est mise en œuvre, qui consiste à quantifier la prépondérance du signal par rapport à une ligne de base définie sur plusieurs centaines de millisecondes, avant et après chaque évènement détecté. Au terme de cette étape subséquente, les évènements ayant une similarité statistique trop proche du modèle de la ligne de base sont rejetés. Finalement, une dernière étape consiste à extraire différents paramètres des évènements sélectionnés, effectuer une réduction de dimensionnalité, puis à regrouper ces évènements de manière non supervisée, en quatre groupes.

Les données sont filtrées dans une large bande de fréquence. L'impact du spectre en $1/f^\alpha$ est toujours très

important car la bande du filtre, comprise entre 80 et 500 Hz, est très large. Une oscillation haute fréquence, notamment supérieure à 250 Hz, devra être très puissante pour pouvoir passer le premier seuil. De nombreuses oscillations peuvent être manquées. De plus, la première étape sélectionne tous les transitoires pointus, qui sont ensuite regroupés. Un inconvénient majeur est que cette méthode ne gère pas les évènements complexes, c'est-à-dire des évènements de types différents ayant lieu en même temps. Or, les oscillations hautes fréquences surmontent presque toujours une pointe épileptique. Ainsi, la méthode décrite dans ce document apparaît manquer de nombreuses oscillations hautes fréquences. On notera que la définition de la ligne de base de cette technique suppose que les évènements d'intérêts sont éloignés dans le temps, d'au moins 600 ms environ, ce qui n'est pas toujours le cas. Ces périodes avant et après l'événement ne correspondent pas nécessairement à une ligne de base.

Le document intitulé "Human Intracranial High Frequency Oscillations (HFOs) Detected by Automatic Time-Frequency Analysis", Burnos et al., Plos One, avril 2014, vol. 9, décrit un détecteur d'oscillations hautes fréquences qui met en œuvre une première étape similaire à celle divulguée dans le document précité, publié sous le numéro US2012/0245481A1, à savoir l'application de l'algorithme de détection de Staba et al., 2002. Selon ce document, les données sont tout d'abord filtrées entre 80 et 500Hz puis un seuil est appliqué. Par contre, le seuil n'est pas appliqué sur une mesure d'énergie sur une courte période temporelle mais sur l'enveloppe du signal filtré. Il s'agit de la transformation d'Hilbert. Dans un souci de limiter les fausses détections, une seconde étape est mise en œuvre. Un temps-fréquence est calculé en utilisant la transformée de Stockwell autour des évènements d'intérêt. Puis, la densité spectrale de puissance instantanée est mesurée dans une plus petite fenêtre de temps. Finalement, trois points du spectre obtenu sont sélectionnés : le maximum local dans la bande 60-500 Hz (High Frequency Peak - HiFP), le minimum/creux entre 40 Hz et la fréquence du HiFP et le maximum local le plus proche du creux dans les basses fréquences. Un évènement est alors qualifié d'oscillation haute fréquence si la puissance mesurée au HiFP est bien supérieure à celle mesurée dans le creux et à celle mesurée à pic basse fréquence (Low Frequency Peak - LoFP).

Le signal est filtré entre 80 et 500 Hz. Le temps-fréquence n'est pas utilisé en tant que tel puisque qu'il y a un retour à l'analyse du spectre pour décider s'il y a un oscillation haute fréquence ou non. À noter qu'il ne mentionne à aucun moment la possibilité de détecter des pointes surmontées d'oscillations hautes fréquences.

## RESUME DE L'INVENTION

Compte tenu de ce qui précède, un problème technique que se propose de résoudre l'invention est de fournir un procédé de détection d'éléments d'intérêt dans des signaux électrophysiologiques, ainsi qu'un détecteur pour la mise en œuvre d'un tel procédé, qui pallie les inconvénients précités de l'art antérieur, et qui, en particulier, transforme l'activité de fond en un bruit blanc pour équilibrer la puissance des activités à travers les fréquences, et qui permette une représentation et une détection adéquate des oscillations basses et hautes fréquences et des pointes ou transitoires, tout en conservant les informations qui leur sont associées telles que leur fréquence, la détection étant effectuée même si les évènements ont lieu en même temps ou à des temps voisins, sans *a priori* sur les bandes de fréquences concernées.

La solution proposée de l'invention à ce problème technique a pour premier objet un procédé de détection automatique d'éléments d'intérêt dans des signaux électrophysiologiques comprenant les étapes, mises en œuvre par un ordinateur, selon lesquelles : on fournit des signaux électrophysiologiques ; on réalise une représentation temps-fréquence blanchie desdits signaux électrophysiologiques ; on établit un seuil ; on applique ce seuil à la représentation temps-fréquence blanchie ; on détecte, dans la représentation temps-fréquence blanchie, des maxima locaux qui sont supérieurs ou égaux au seuil appliqué, et selon lequel procédé, pour la réalisation de la représentation temps-fréquence blanchie, on applique une transformée en ondelettes continue et on calcule le module au carré des coefficients d'ondelettes après avoir normalisé leurs parties réelle et imaginaire, un facteur de normalisation étant calculé pour chaque fréquence en ajustant un modèle de bruit gaussien sur la partie centrale de l'histogramme des coefficients réels.

Elle a pour second objet un produit programme d'ordinateur comprenant des instructions qui, lorsque le programme est exécuté par un ordinateur, conduisent celui-ci à mettre en œuvre le procédé ci-dessus.

De manière avantageuse, - les signaux électrophysiologiques sont des signaux intracrâniens ; - les signaux intracrâniens sont des signaux stéréo-électroencéphalographiques ; - les éléments d'intérêt sont des oscillations basses et hautes fréquences et des pointes ; - la transformée en ondelettes continue est calculée à partir de la formule suivante :

$$T_f(b,a) = \frac{1}{\sqrt{a}} \int_{-\infty}^{+\infty} f(t) \overline{\psi\left(\frac{t-b}{a}\right)} dt$$

dans laquelle $f$ est le signal électrophysiologique, $T$ est la transformée en ondelettes continue, $\psi$ est l'ondelette, $a$ le facteur de dilatation, $b$ le facteur de translation et $t$ est le temps ; - l'ondelette choisie est une ondelette de dérivée gaussienne (DoG), analytique et son expression dans le domaine fréquentiel est la suivante : $\tilde{\psi}(f) = f^n \exp(-f^2)$ pour $f \geq 0$ et $= 0$ pour $f < 0$ dans laquelle $f$ est la fréquence, n est l'ordre de la dérivée et $\tilde{\psi}$ est la transformée de Fourier de l'ondelette ; - le facteur de normalisation est calculé pour chaque fréquence en ajustant un modèle de bruit gaussien sur la partie centrale de l'histogramme des coefficients réels ; - le seuil est défini par la formule suivante :

$$thr = x \quad | \quad \mathrm{lFDR}(x) = \mathrm{H}_0(x)/\mathrm{H}_G(x) < Q$$

dans laquelle *thr* est le seuil, Q est le taux d'erreur acceptable, $H_o$ est l'hypothèse nulle et $H_G$ est la distribution totale ; - le procédé comprend en outre une étape de détermination de l'étendue temporelle et fréquentielle des maxima locaux ; - le procédé comprend en outre une étape de classification des éléments d'intérêt en tant que transitoire ou oscillation ; - le procédé comprend en outre une étape de visualisation des éléments d'intérêts dans le domaine temps-fréquence et/ou le domaine temporel ; - les signaux électrophysiologiques sont des signaux électrophysiologiques de patients épileptiques ; et - le module logiciel comporte un classifieur.

BREVE DESCRIPTION DES FIGURES

L'invention sera mieux comprise à la lecture de la description non limitative qui suit, rédigée au regard des dessins annexés, dans lesquels :

les figures 1A à 1C sont des histogrammes qui montrent les distributions de la partie réelle des coefficients d'ondelettes à différentes fréquences, sans normalisation ou avec une normalisation et notamment avec une normalisation selon le procédé selon l'invention ;

les figures 2A et 2B montrent chacune un exemple d'oscillations hautes fréquences enregistrées chez un patient épileptique en réalisant une représentation temps-fréquence blanchie selon le procédé selon l'invention ;

la figure 3 illustre l'application du IFDR pour la mise en œuvre du procédé selon l'invention ;

les figures 4A et 4B illustrent deux exemples de détection selon le procédé selon l'invention dans le domaine temporel avec le signal et sa reconstruction blanchie (première et deuxième ligne, repspectivement) ainsi que dans le domaine temps-fréquence blanchi (troisième ligne) ;

les figures 5A et 5B représentent les détections dans l'espace des paramètres et la répartition des fréquences des oscillations selon l'invention ; et

les figures 6A et 6B montrent un exemple d'implémentation du détecteur et de la visualisation simultanée du signal dans le domaine temporel avec le signal d'origine et sa reconstruction blanchie ainsi que dans le domaine temps-fréquence blanchi, dans l'utilisation clinique quotidienne selon l'invention.

DESCRIPTION DETAILLEE DE L'INVENTION

Le procédé de détection selon l'invention est un procédé de représentation et de détection automatique d'éléments d'intérêt dans des signaux électrophysiologiques. Ces signaux sont des tracés biologiques/physiques, quels que soit leur origine.

Dans une première étape du procédé selon l'invention, on fournit des signaux électrophysiologiques. Ces signaux sont des signaux intracrâniens et, notamment, des signaux stéréo-électroencéphalographiques (SEEG) notés *f*. Il s'agit de signaux complexes comprenant des oscillations basses et hautes fréquences et des pointes ou transitoires. Ces oscillations, pointes et transitoires sont les éléments/événements d'intérêt qui sont détectés selon le procédé selon l'invention.

En vue de cette détection, on réalise une représentation temps-fréquence blanchie desdits signaux électrophysiologiques. Autrement dit, on redresse les signaux tout en conservant un bon rapport signal à bruit pour les éléments d'intérêt.

Le blanchiment est réalisé dans le domaine temps-fréquence puis appliqué au domaine temporel pour la visualisation.

Pour la réalisation de la représentation temps-fréquence blanchie, on applique une transformée en ondelettes continue (CWT - Continuous Wavelet Transform) notée *T* auxdits signaux. Cette transformée *T* se calcule à partir de la formule suivante :

$$T_f(b,a) = \frac{1}{\sqrt{a}} \int\limits_{-\infty}^{+\infty} f(t)\overline{\psi\left(\frac{t-b}{a}\right)}dt$$

dans laquelle $\psi$ est l'ondelette, $\alpha$ le facteur de dilatation, $b$ le facteur de translation et t est le temps. L'ondelette choisie est une ondelette de dérivée gaussienne (DoG), analytique. Ses propriétés analytiques permettent de reconstruire le signal, contrairement aux ondelettes de Morlet, et ainsi d'obtenir le signal blanchi dans le domaine temporel ainsi que cela sera précisé dans la suite de la présente description selon l'invention. Son expression dans le domaine fréquentiel est la suivante :

$$\tilde{\psi}(f) = f^n \exp(-f^2) \text{ pour } f \geq 0 \text{ et } = 0 \text{ pour } f < 0$$

Une fois les coefficients d'ondelettes obtenus, on les normalise pour permettre de mieux faire ressortir les éléments d'intérêt et, notamment, les oscillations hautes fréquences. Cela correspond à une étape selon l'invention selon laquelle on normalise la représentation temps-fréquence des signaux, pour obtenir une représentation blanchie/normalisée.

La normalisation choisie est une normalisation dite $Z_{H0}$ ou $H_0$ Z-score. Cette normalisation permet une représentation optimale des oscillations hautes fréquences, sans diminuer les rapports signal sur bruit ou perdre le contenu des basses fréquences. Contrairement aux méthodes classiques, la puissance de l'activité de fond à chaque fréquence est estimée directement sur les données d'intérêt et ne requière pas la difficile définition d'une ligne de base. Concrètement, un modèle de bruit gaussien est ajusté sur la partie centrale de l'histogramme des coefficients réels à chaque fréquence. Puis les coefficients sont transformés en z par la formule suivante : $T_{f,z_{H_0}}^i[n,m] = \frac{T_f^i[n,m]-\mu[m]}{\sigma[m]}$ où n et m sont respectivement les indices temporel et fréquentiel, $T_{f,z_{H_0}}^i[n,m]$ et $T_f^i[n,m]$ sont le coefficient i (réel ou imaginaire) pour les indice n et m après et avant normalisation, $\mu[m]$ et $\sigma[m]$ correspondent à la moyenne et l'écart-type de la gaussien estimée à la fréquence d'indice m. La normalisation transforme l'activité de fond en un bruit blanc pour équilibrer la puissance des activités à travers les fréquences. Le spectre est donc blanchi. De plus, elle rend possible la représentation optimale du signal blanchi à la fois dans le domaine temps-fréquence et dans le domaine temporel. Cette normalisation met à la même échelle toutes les distributions des coefficients réels et imaginaires par fréquence.

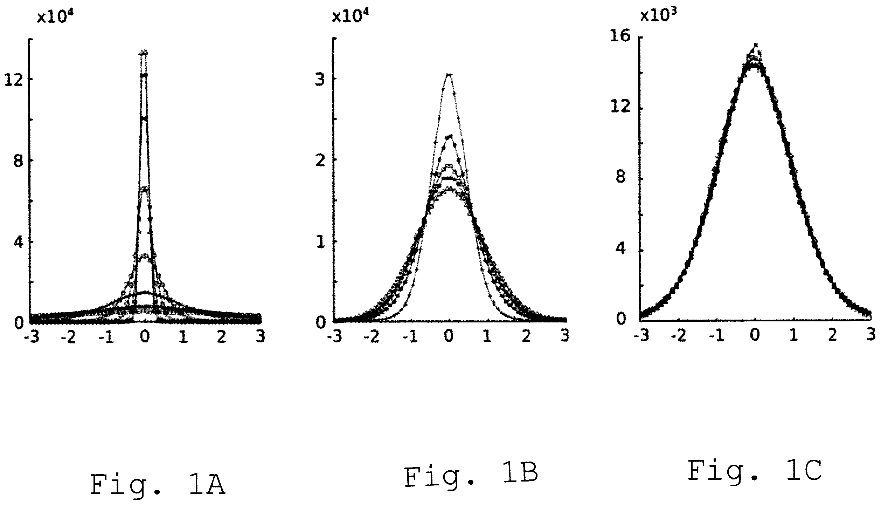

Aux figures 1A à 1C, on a représenté des histogrammes qui montrent, pour différentes fréquences, les distributions de la partie réelle des coefficients d'ondelettes à différentes échelles, sans normalisation ou avec une normalisation et, notamment, avec la normalisation $Z_{H0}$. A la figure 1A, les distributions de la partie réelle des coefficients d'ondelettes ne sont pas normalisées. On note une grande différence de largeur entre les distributions. A la figure 1B, les distributions sont normalisées avec la moyenne et l'écart type - $\mu[\alpha]$ et $\sigma[\alpha]$ - estimés sur toute les distributions. On note toujours une différence entre les largeurs des distributions. A la figure 1C, les distributions sont normalisées au moyen de la normalisation précitée $Z_{H0}$. Cette fois, toutes les distributions ont la même étendue au travers des fréquences.

Pour obtenir la représentation temps-fréquence blanchie, on calcule, selon l'invention, le module au carré des coefficients d'ondelettes après avoir normalisé leurs parties réelle et imaginaire.

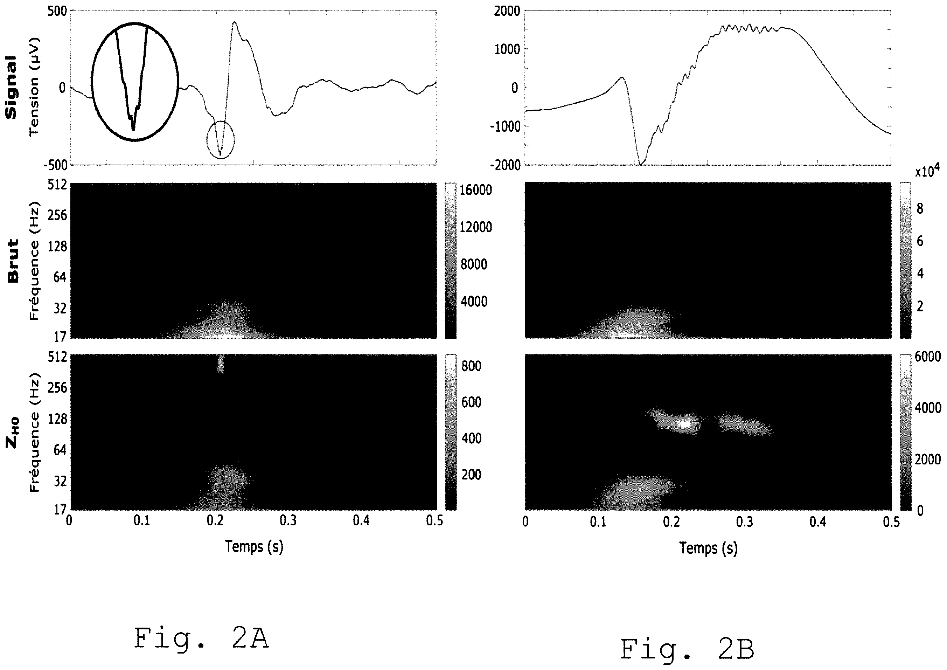

Les figures 2A et 2B montrent chacune un exemple d'oscillations hautes fréquences enregistrées chez un patient épileptique. Pour chaque exemple, la ligne du haut montre un signal comprenant des éléments intérêts dans le domaine temporel, la ligne intermédiaire montre la représentation temps-fréquence brute associée, et la ligne du bas montre la représentation temps-fréquence blanchie au moyen de la normalisation dite $Z_{H0}$. A la figure 2A, les oscillations sont des oscillations FR. A la figure 2B, les oscillations sont des oscillations HG. Sur la ligne du haut de la figure 2A, l'insert correspond à un zoom sur les oscillations hautes fréquences FR. Il apparait que les oscillations hautes fréquences sont difficiles à identifier sur les lignes du haut et les lignes intermédiaires. Cependant, elles apparaissent clairement sur la ligne du bas, grâce à la normalisation $Z_{H0}$.

Comme la distribution de la partie réelle des coefficients est identique à travers les fréquences, il est possible de les étudier en même temps et d'appliquer un seuil unique basé sur le taux local de fausses découvertes (lFDR - local False Discovery Rate Efron 2005). On établit donc, selon une autre étape selon l'invention, un seuil.

Le lFDR est une approche bayésienne empirique qui suppose que le bruit $H_0$ compose la majeure partie du centre de la distribution $H_G$ et que le reste de la distribution $H_1$ est produite par le signal d'intérêt. Le seuil est défini la formule suivante :

$$thr = x \quad | \quad \mathrm{IFDR}(x) = \mathrm{H}_0(x)/\mathrm{H}_G(x) < Q$$

dans laquelle *thr* est le seuil, Q est le taux d'erreur acceptable, $H_o$ est l'hypothèse nulle et $H_G$ est la distribution totale.

En pratique, on obtient deux seuils. Il s'agit d'un premier seuil *thr⁻* pour la partie négative et d'un second seuil *thr⁺* pour la partie positive.

Le seuil de l'IFDR est :

$$thr_{\mathrm{IFDR}} = \frac{thr^+ - thr^-}{2}$$

En étudiant les histogrammes des coefficients réels de l'activité de fond humain (Real Human Background, BKG), on remarque que ces distributions sont décrites par une gaussienne. La partie centrale de l'histogramme des $T^i_{f,z_{H0}}$ pris ensemble peut alors être modélisée par une gaussienne centrée réduite et le taux d'erreur Q est laissé à l'appréciation de l'utilisateur.

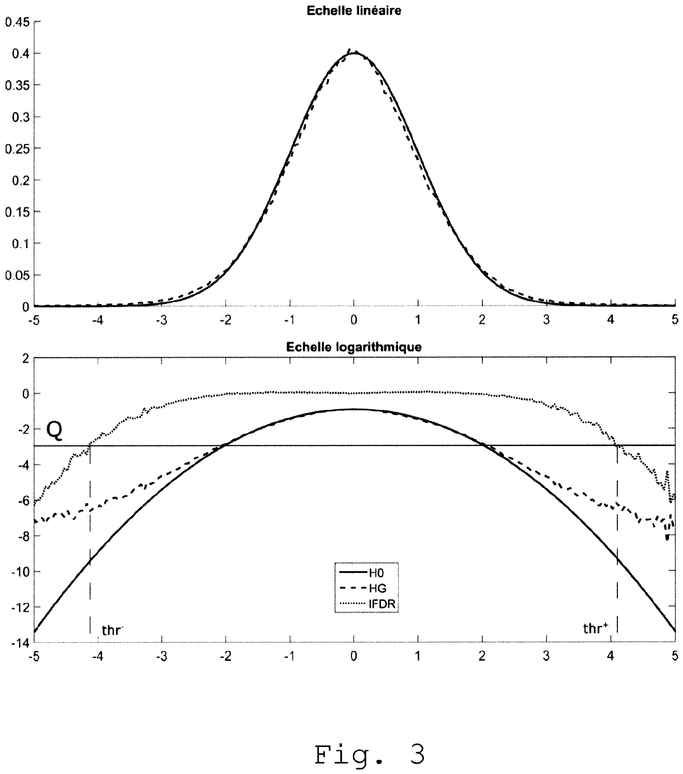

Une illustration de l'application du IFDR est montrée à la figure 3. Dans cette figure, la distribution totale $H_G$, l'hypothèse nulle $H_0$ ainsi que le IFDR sont représentés en traits tirés, en noirs pleins et en pointillés, respectivement. Le seuil sur le taux d'erreur Q est représenté par un trait plein horizontal. Les seuils thr+ et thr- sont obtenus à l'intersection du IFDR et de Q.

Selon une autre étape du procédé selon l'invention, on applique le seuil à la représentation temps-fréquence normalisée. A cet effet, le seuil obtenu grâce au IFDR est élevé au carré :

$$thr_{z_{H_0}} = thr^2_{\mathrm{IFDR}}$$

Il est alors possible de détecter, dans la représentation temps-fréquence normalisée, des maxima locaux qui sont supérieurs ou égaux au seuil appliqué, et qui correspondent à des éléments d'intérêt qui ressortent du bruit. Grâce au blanchiment des données et à l'étude des évènements en temps-fréquence, les pointes et les oscillations sont détectées, qu'elles aient lieux en même temps ou non.

En pratique, cette détection consiste à sélectionner tous les maxima locaux qui sont plus élevés que le seuil établi par le IFDR. Les maxima locaux sont relatifs aux évènements localisés à la fois en temps et en fréquence comme le sont les oscillations recherchées. On peut alors avoir le temps d'occurrence ainsi que la fréquence d'oscillation. Les pointes épileptiques elles aussi sont bien localisées en temps-fréquence, mais sont plus étalées en fréquence et moins étalée en temps que les oscillations et leur maximum local est plus bas que la bande de fréquence des oscillations hautes fréquences. A l'inverse, les artéfacts de type Dirac ne produisent pas de maxima locaux et donc ne sont pas détectés. En réalité, un artéfact mélangé avec du bruit ou un transitoire très bref avec une oscillation peuvent parfois créer des maxima locaux erronés à haute fréquence. Cependant, comme les détections se font dans le cadre théorique des ondelettes, on peut comparer la largeur de la tache/ de l'îlot (blob) relatif au maximum local avec la largeur théorique de l'îlot qui aurait été générée par un pic de Dirac. Cela permet de différencier un élément bref (c'est-à-dire une pointe épileptique ou un transitoire bref) par rapport à une oscillation quelle que soit sa fréquence. De plus, comme la largeur fréquentielle des ondelettes est constante en échelle logarithmique, on peut aussi distinguer les oscillations qui ont toujours une largeur fréquentielle bornée quelles que soient leur fréquence, des transitoires qui ont une largeur fréquentielle plus étendue.

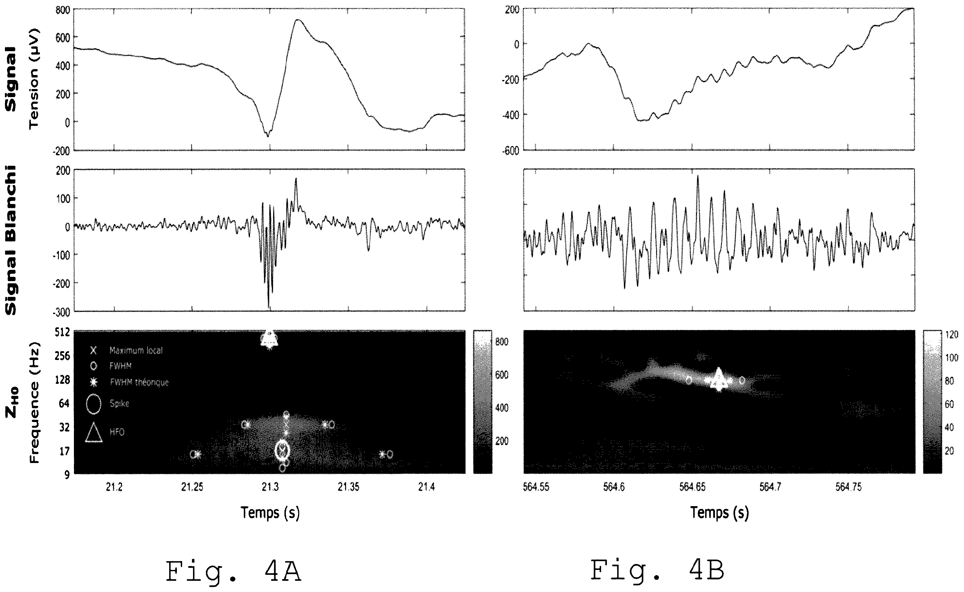

Deux exemples de détection sont illustrés aux figures 4A et 4B. Dans cette figure, les croix correspondent aux maxima locaux supérieurs au seuil, les cercles blancs à la largeur à mi-hauteur mesurée (Full Width at Half Maximum, FWHM), les astérisques à la largeur à mi-hauteur théorique, le grand cercle à une pointe épileptique (spike) et le triangle à une oscillation hautes fréquences. Il apparaît que la largeur temporelle théorique est très proche de la largeur temporelle mesurée pour une pointe.

Le procédé selon l'invention permet donc de visualiser et d'identifier plusieurs types d'activités physiologiques comme les oscillations hautes fréquences, les pointes épileptiques et les oscillations de plus basse fréquence. Les paramètres utilisés pour classer les événements ne dépendent pas des fréquences. Grâce à ce procédé, l'identification des zones cérébrales produisant des oscillations hautes fréquences est facilitée, car automatique.

On notera que les étapes combinées de normalisation et du IFDR permettent de différencier l'activité de fond des éléments d'intérêt et assurent leur authenticité. La combinaison de ces deux étapes permet aussi de détecter les oscillations hautes fréquences qui auraient été rejetées car non visibles dans le signal d'origine. De plus, chaque oscillation sera étiquetée par une fréquence. On peut ainsi déterminer les bandes de fréquences physiologiques et pathologiques pour les patients ce qui n'était pas possible avec les autres détecteurs. Il est à noter que le procédé ne présuppose pas un découpage en bandes de fréquences.

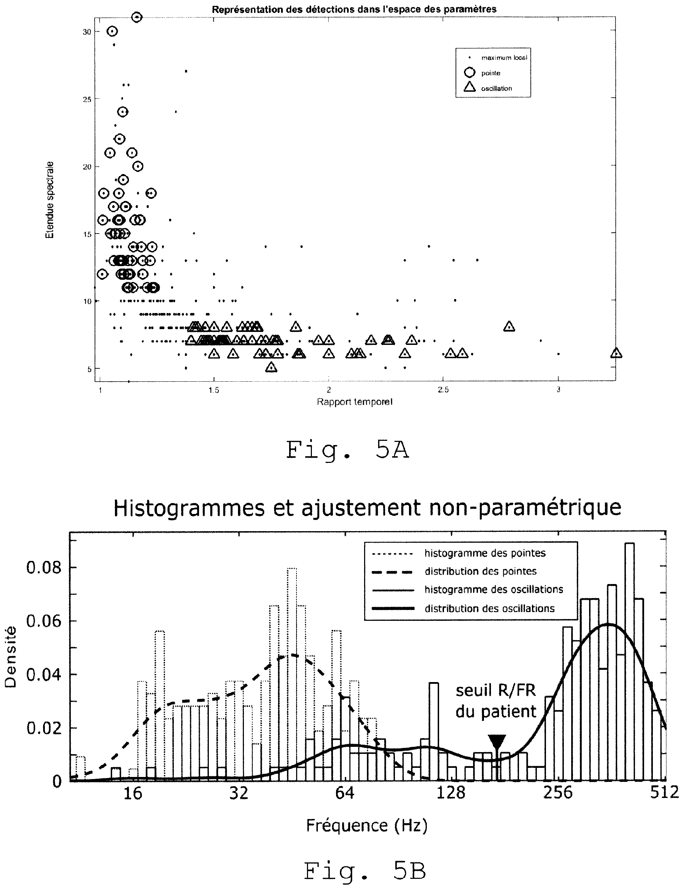

A la figure 5A, toutes les détections sont représentées par un point de coordonnées, leur étendue fréquentielle et leur rapport temporel. Un seuil sur la limite de durée, équivalent au nombre d'oscillations, et sur l'étendue spectrale permet de séparer les oscillations des pointes épileptiques et des évènements non oscillants, c'est-à-dire comprenant moins de 3 ou 4 oscillations. Les oscillations, les pointes épileptiques et les évènements non oscillants sont illustrés par des points. Les détections sélectionnées comme pointes et comme oscillations sont respectivement représentées par des cercles et des triangles.

Les histogrammes des fréquences des pointes et des oscillations détectées sont représentés à la figure 5B. Un ajustement non-paramétrique est effectué en trait tiré pour les pointes et en trait plein noir pour les oscillations. On remarque que la zone étudiée produit beaucoup plus d'oscillations hautes fréquences que d'oscillations basses fréquences. Grâce à cette représentation, on se rend compte qu'il serait plus intéressant de placer la coupure des oscillations R/FR à 170 Hz plutôt que de prendre comme repère les bandes R et FR définies de manière conventionnelle.

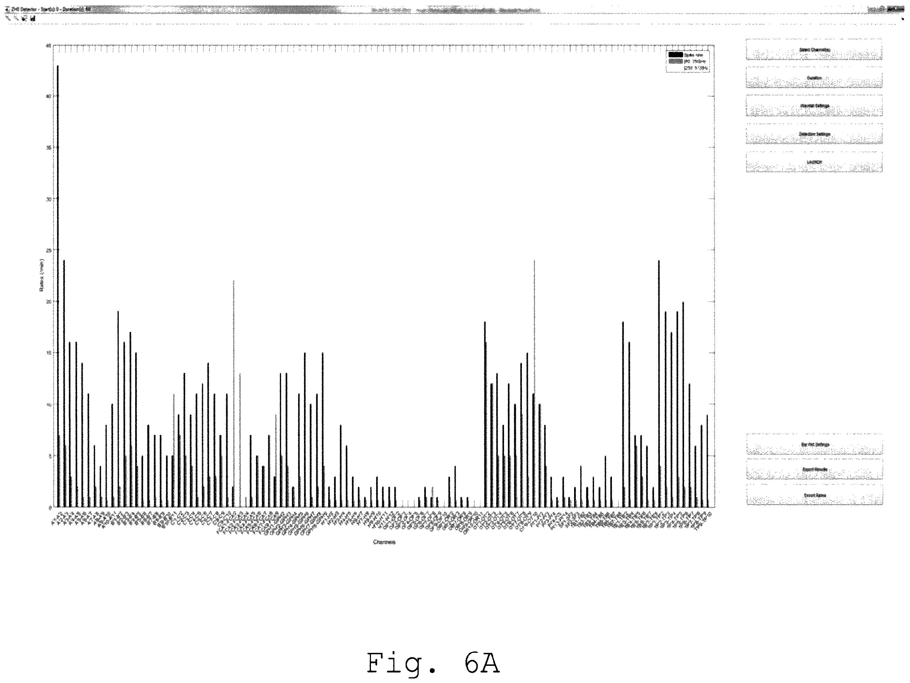

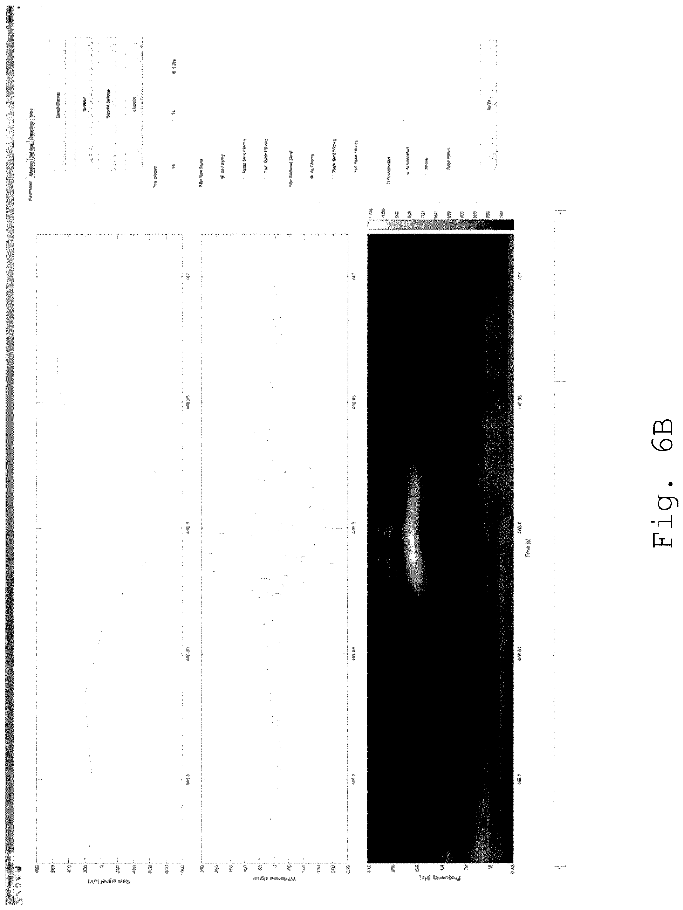

Le détecteur selon l'invention peut être implémenté dans un logiciel tel que le logiciel AnyWave™ sous forme de module d'extension (plugin) pour une utilisation clinique régulière. Cette plate-forme logicielle est décrite par exemple dans le document intitulé « AnyWave: a cross-platform and modular software for visualizing and processing electrophysiological signals », Colombet et al., Journal Neurosciences Methods, mars 2015, 242, 118-26. Le plugin est composé de deux éléments, une interface (figure 6A) permettant de sélectionner plusieurs canaux des différentes électrodes et de lancer le détecteur ainsi que de représenter les résultats sous forme de diagramme des taux de détection des pointes épileptiques et des oscillations par bandes de fréquences choisies par l'utilisateur, et une seconde interface (figure 6B) permettant de visualiser le signal d'un canal de manière synchrone dans le domaine temporel et dans le domaine temps-fréquence normalisé et d'afficher le signal au temps de détection obtenu précédemment : visualisation simultanée du signal dans le domaine temporel avec le signal d'origine et sa reconstruction blanchie ainsi que dans le domaine temps-fréquence blanchi. Chaque plugin communique avec le logiciel pour acquérir les signaux et leurs informations. Le logiciel comprenant le plugin est destiné à être mis en œuvre par une station de travail, éventuellement en lien avec un serveur, dans un environnement informatique classique du type Windows™. La station de travail comprend au moins une mémoire dans laquelle sera enregistré le logiciel, un processeur pour son exécution, et un écran pour l'affichage des résultats. La détection est automatique. La mise en œuvre du procédé ne requiert pas la présence d'une personne qualifiée sur le plan médical.

## Revendications

1. Procédé de détection automatique d'éléments d'intérêt dans des signaux électrophysiologiques comprenant les étapes, mises en œuvre par un ordinateur, selon lesquelles :

   on fournit des signaux électrophysiologiques ;
   on réalise une représentation temps-fréquence blanchie desdits signaux électrophysiologiques ;
   on établit un seuil ;
   on applique ce seuil à la représentation temps-fréquence blanchie ;
   on détecte, dans la représentation temps-fréquence blanchie, des maxima locaux qui sont supérieurs ou égaux au seuil appliqué,
   et selon lequel procédé, pour la réalisation de la représentation temps-fréquence blanchie, on applique une transformée en ondelettes continue et on calcule le module au carré des coefficients d'ondelettes après avoir normalisé leurs parties réelle et imaginaire, un facteur de normalisation étant calculé pour chaque fréquence en ajustant un modèle de bruit gaussien sur la partie centrale de l'histogramme des coefficients réels.

2. Procédé selon la revendication 1, **caractérisé en ce que** les signaux électrophysiologiques sont des signaux intracrâniens.

3. Procédé selon la revendication 2, **caractérisé en ce que** les signaux intracrâniens sont des signaux stéréo-électroencéphalographiques.

4. Procédé selon l'une des revendications 1, 2 ou 3, **caractérisé en ce que** les éléments d'intérêt sont des oscillations basses et hautes fréquences et des pointes.

**5.** Procédé selon la revendication 4, **caractérisé en ce que** la transformée en ondelettes continue est calculée à partir de la formule suivante :

$$T_f(b, a) = \frac{1}{\sqrt{a}} \int_{-\infty}^{+\infty} f(t)\overline{\psi\left(\frac{t-b}{a}\right)} dt$$

dans laquelle f est le signal électrophysiologique, T est la transformée en ondelettes continue, $\psi$ est l'ondelette, $\alpha$ le facteur de dilatation, $b$ le facteur de translation et $t$ est le temps.

**6.** Procédé selon l'une des revendications 4 ou 5, **caractérisé en ce que** l'ondelette choisie est une ondelette de dérivée gaussienne (DoG), analytique et son expression dans le domaine fréquentiel est la suivante :

$$\tilde{\psi}(f) = f^n \exp(-f^2) \text{ pour } f \geq 0 \text{ et } = 0 \text{ pour } f < 0$$

dans laquelle $f$ est la fréquence, n est l'ordre de la dérivée et $\tilde{\psi}$ est la transformée de Fourier de l'ondelette.

**7.** Procédé selon l'une des revendications précédentes, **caractérisé en ce que** le seuil est défini par la formule suivante :

$$thr = \tilde{x} \mid \mathrm{IFDR}(x) = \mathrm{H}_0(x)/\mathrm{H}_G(x) < Q$$

dans laquelle $thr$ est le seuil, Q est le taux d'erreur acceptable, $H_o$ est l'hypothèse nulle et $H_G$ est la distribution totale.

**8.** Procédé selon l'une des revendications précédentes, **caractérisé en ce qu'**il comprend en outre une étape de détermination de l'étendue temporelle et fréquentielle des maxima locaux.

**9.** Procédé selon l'une des revendications précédentes, **caractérisé en ce qu'**il comprend en outre une étape de classification des éléments d'intérêt en tant que transitoire ou oscillation.

**10.** Procédé selon l'une des revendications précédentes, **caractérisé en ce qu'**il comprend en outre une étape de visualisation des éléments d'intérêts.

**11.** Procédé selon l'une des revendications précédentes, **caractérisé en ce que** les signaux électrophysiologiques sont des signaux électrophysiologiques de patients épileptiques.

**12.** Produit programme d'ordinateur comprenant des instructions qui, lorsque le programme est exécuté par un ordinateur, conduisent celui-ci à mettre en œuvre le procédé selon l'une des revendications précédentes..

**Patentansprüche**

**1.** Verfahren zur automatischen Erkennung von Elementen von Interesse in elektrophysiologischen Signalen, das die Schritte umfasst, die von einem Computer umgesetzt werden, wonach:

elektrophysiologische Signale bereitgestellt werden;
eine bereinigte Zeit-Frequenz-Darstellung der elektrophysiologischen Signale realisiert wird;
eine Schwelle erstellt wird;
diese Schwelle auf die bereinigte Zeit-Frequenz-Darstellung angewendet wird;
in der bereinigten Zeit-Frequenz-Darstellung lokale Maxima erkannt werden, die größer oder gleich der angewendeten Schwelle sind,

und entsprechend dem Verfahren, zum Realisieren der bereinigten Zeit-Frequenz-Darstellung, eine kontinuierliche Wavelet-Transformation angewendet wird,

und das Quadratmodul der Wavelet-Koeffizienten berechnet wird, nachdem deren reellen und imaginären Teile normiert worden sind, wobei ein Normierungsfaktor für jede Frequenz berechnet wird, indem ein Gauß'sches Rauschmodell im zentralen Teil des Histogramms der reellen Koeffizienten angepasst wird.

2. Verfahren nach Anspruch 1, **dadurch gekennzeichnet, dass** die elektrophysiologischen Signale intrakranielle Signale sind.

3. Verfahren nach Anspruch 2, **dadurch gekennzeichnet, dass** die intrakraniellen Signale stereoelektroenzephalographische Signale sind.

4. Verfahren nach einem der Ansprüche 1, 2 oder 3, **dadurch gekennzeichnet, dass** die Elemente von Interesse Nieder- und Hochfrequenzoszillationen und Spitzen sind.

5. Verfahren nach Anspruch 4, **dadurch gekennzeichnet, dass** die kontinuierliche Wavelet-Transformation anhand der folgenden Formel berechnet wird:

$$T_f(b, a) = \frac{1}{\sqrt{a}} \int\limits_{-\infty}^{+\infty} f(t) \overline{\psi\left(\frac{t-b}{a}\right)} dt$$

wobei f das elektrophysiologische Signal ist, T die kontinuierliche Wavelet-Transformation ist, $\psi$ das Wavelet ist, $\alpha$ der Ausdehnungsfaktor ist, *b der* Translationsfaktor ist und *t* die Zeit ist.

6. Verfahren nach einem der Ansprüche 4 oder 5, **dadurch gekennzeichnet, dass** das ausgewählte Wavelet ein Wavelet einer analytischen Gaußableitung (DoG) ist und dessen Ausdruck in der Frequenzdomäne der Folgende ist:

$$\overline{\psi}(f) = f^n \exp(-f^2) \text{ für } f \geq 0 \text{ und } = 0 \text{ für } < 0$$

wobei *f* die Frequenz ist, n die Ableitungsordnung ist und $\tilde{\psi}$ die Fourier-Transformation des Wavelet ist.

7. Verfahren nach einem der vorstehenden Ansprüche, **dadurch gekennzeichnet, dass** die Schwelle durch die folgende Formel definiert wird:

$$thr = x \quad | \quad \text{IFDR}(x) = \text{H}_0(x)/\text{H}_G(x) < Q$$

wobei *thr* die Schwelle ist, Q die zulässige Fehlerquote ist, *Ho* die Nullhypothese ist, und $\text{H}_G$ die Gesamtverteilung ist.

8. Verfahren nach einem der vorstehenden Ansprüche, **dadurch gekennzeichnet, dass** es weiter einen Schritt des Bestimmens der Zeit- und Frequenzausdehnung der lokalen Maxima umfasst.

9. Verfahren nach einem der vorstehenden Ansprüche, **dadurch gekennzeichnet, dass** es weiter einen Schritt der Einstufung der Elemente von Interesse als transitorisch oder Oszillation umfasst.

10. Verfahren nach einem der vorstehenden Ansprüche, **dadurch gekennzeichnet, dass** es weiter einen Visualisierungsschritt der Elemente von Interesse umfasst.

11. Verfahren nach einem der vorstehenden Ansprüche, **dadurch gekennzeichnet, dass** die elektrophysiologischen Signale elektrophysiologische Signale von Epilepsiepatienten sind.

12. Computerprogrammprodukt, Anweisungen umfassend, die, wenn das Programm durch einen Computer ausgeführt wird, diesen dazu führen, das Verfahren nach einem der vorstehenden Ansprüche umzusetzen.

**Claims**

1. A method for automatically detecting elements of interest in electrophysiological signals comprising the steps, implemented by a computer, according to which:

   electrophysiological signals are provided;
   a whitened time-frequency representation is performed from said electrophysiological signals;
   a threshold is established;
   this threshold is applied to the whitened time-frequency representation;
   in the whitened time-frequency representation, local maxima that are greater than or equal to the applied threshold are detected,
   and according to which method, for performing the whitened time-frequency representation, a continuous wavelet transform is applied and the squared modulus of the wavelet coefficients is calculated after having normalised their real and imaginary parts, a normalisation factor being calculated for each frequency by adjusting a Gaussian noise model on the central part of the histogram of the real coefficients.

2. The method according to claim 1, **characterised in that** the electrophysiological signals are intracranial signals.

3. The method according to claim 2, **characterised in that** the intracranial signals are stereoelectroencephalographic signals.

4. The method according to one of claims 1, 2 or 3, **characterised in that** the elements of interest are low and high frequency oscillations and tips.

5. The method according to claim 4, **characterised in that** the continuous wavelet transform is calculated from the following formula:

$$T_f(b, a) = \frac{1}{\sqrt{a}} \int_{-\infty}^{+\infty} f(t) \overline{\psi\left(\frac{t-b}{a}\right)} \, dt$$

   wherein f is the electrophysiological signal, T is the continuous wavelet transform, $\psi$ is the wavelet, $\alpha$ the expansion factor, b the translation factor and t is the time.

6. The method according to one of claims 4 or 5, **characterised in that** the selected wavelet is an analytical Gaussian derivative wavelet (DoG), and its expression in the frequency domain is as follows:

$$\overline{\psi}(f) = f^n \exp(-f^2) \text{ for } f \geq 0 \text{ } and \text{ } = 0 \text{ for } < 0$$

   wherein f is the frequency, n is the order of the derivative and $\overline{\psi}$ is the wavelet Fourier transform.

7. The method according to one of the preceding claims, **characterised in that** the threshold is defined by the following formula:

$$thr = \tilde{x} \mid \text{IFDR}(x) = \text{H}_0(x)/\text{H}_G(x) < Q$$

   wherein thr is the threshold, Q is the acceptable error rate, $H_o$ is the null hypothesis and $H_G$ is the total distribution.

8. The method according to one of the preceding claims, **characterised in that** it further comprises a step of determining the time and frequency extent of local maxima.

9. The method according to one of the preceding claims, **characterised in that** it further comprises a step of classifying the elements of interest as transient or oscillation.

10. The method according to one of the preceding claims, **characterised in that** it further comprises a step of visualising the elements of interest.

11. The method according to one of the preceding claims, **characterised in that** the electrophysiological signals are electrophysiological signals of epileptic patients.

12. A computer program product comprising instructions which, when the program is executed by a computer, lead the latter to implement the method according to one of the preceding claims.

Fig. 1A          Fig. 1B          Fig. 1C

Fig. 2A          Fig. 2B

Fig. 3

Fig. 4A          Fig. 4B

EP 3 422 935 B1

Fig. 5A

## Histogrammes et ajustement non-paramétrique

Fig. 5B

Fig. 6B

## RÉFÉRENCES CITÉES DANS LA DESCRIPTION

**Documents brevets cités dans la description**

- US 20120245481 A1

**Littérature non-brevet citée dans la description**

- Comparison of spectral analysis methods for characterizing brain oscillations. **VAN VUGT M K et al.** JOURNAL OF NEUROSCIENCE METHODS. ELSEVIER SCIENCE PUBLISHER B.V, 15 Mai 2007, vol. 162, 49-63

- **BURNOS et al.** Human Intracranial High Frequency Oscillations (HFOs) Detected by Automatic Time-Frequency Analysis. *Plos One,* Avril 2014, vol. 9
- **COLOMBET et al.** AnyWave: a cross-platform and modular software for visualizing and processing electrophysiological signals. *Journal Neurosciences Methods,* Mars 2015, vol. 242, 118-26